(19) Europäisches Patentamt
European Patent Office
Office européen des brevets

(11) EP 2 518 100 B1

# (12) EUROPEAN PATENT SPECIFICATION

(45) Date of publication and mention of the grant of the patent:
**18.02.2015 Bulletin 2015/08**

(21) Application number: **10839438.8**

(22) Date of filing: **22.12.2010**

(51) Int Cl.:
***C08G 81/00*** (2006.01) ***A61L 33/00*** (2006.01)
***C08F 8/34*** (2006.01) ***C08F 16/06*** (2006.01)
***C08F 18/08*** (2006.01) ***C08F 26/10*** (2006.01)
***C08G 77/392*** (2006.01) ***C08G 77/46*** (2006.01)
***C08G 83/00*** (2006.01) ***C08G 85/00*** (2006.01)

(86) International application number:
**PCT/JP2010/073092**

(87) International publication number:
**WO 2011/078208 (30.06.2011 Gazette 2011/26)**

## (54) HYDROPHILIC POLYMER COMPOUND HAVING ANTICOAGULATION EFFECT

HYDROPHILE POLYMERVERBINDUNG MIT GERINNUNGSVERHINDERNDER WIRKUNG

COMPOSÉ POLYMÈRE HYDROPHILE POSSÉDANT UNE ACTIVITÉ ANTICOAGULANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2009 JP 2009292146**

(43) Date of publication of application:
**31.10.2012 Bulletin 2012/44**

(73) Proprietor: **Toray Industries, Inc.**
**Tokyo 103-8666 (JP)**

(72) Inventors:
- **SAKAGUCHI, Hirokazu**
**Otsu-shi**
**Shiga 520-8558 (JP)**
- **SAKAGUCHI, Yuka**
**Otsu-shi**
**Shiga 520-8558 (JP)**
- **TANAHASHI, Kazuhiro**
**Otsu-shi**
**Shiga 520-8558 (JP)**

(74) Representative: **Kador & Partner**
**Corneliusstraße 15**
**80469 München (DE)**

(56) References cited:
**JP-A- 6 219 948** **JP-A- 6 219 949**
**JP-A- 9 313 595** **JP-A- H08 131 536**
**JP-A- 2001 213 984** **JP-A- 2006 291 193**
**JP-A- 2007 181 691** **JP-A- 2007 181 692**
**JP-A- 2009 225 824** **US-A- 3 844 989**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Technical Field

The present invention relates to a hydrophilic polymer compound having anticoagulation effect (anticoagulant activity).

### Background Art

The blood coagulation reaction required for coagulating blood is an extremely complicated reaction in which various blood coagulation factors are involved. It is thought that the primary hemostasis stage in which platelets are involved and in the coagulation thrombus formation stage in which blood coagulation factors such as thrombin are involved to stabilize and strengthen fibrin are particularly important.

The blood coagulation reaction is indispensable to the hemostasis of bleeding due to injury or the like. On the other hand, however, in cases where the blood coagulation reaction proceeds due to contact between the blood and a medical device or a medical material, such as an extracorporeal circulation circuit in hemodialysis, there is a risk that the formed coagulation thrombus increases the circulation pressure or occludes a blood vessel.

To decrease these risks, a method of preventing blood coagulation is known wherein heparin which is an anticoagulant is preliminarily administered to the patient who is to undergo hemodialysis. However, this method has a number of problems in that administration of heparin in excess amount causes side effects, the control of administration dose is complicated, the method cannot be applied to a patient having bleeding tendency, and so on.

Recently, in order to avoid these problems, trials for preventing blood coagulation during the therapy by immobilizing a compound having an anticoagulant activity including heparin on the surface of medical devices or medical materials such as blood circuits and the like, have been reported (Patent Documents 1 to 9).

### Prior Art References

#### Patent Documents

Patent Document 1: Japanese Translated PCT Patent Application Laid-open No. 2003-507082
Patent Document 2: JP 2001-213984 A
Patent Document 3: Japanese Translated PCT Patent Application Laid-open No. 2004-525888
Patent Document 4: JP 2006-291193 A
Patent Document 5: WO 08/032758
Patent Document 6: JP 2009-225824 A
Patent Document 7: JP 2010-082067 A
Patent Document 8: JP 2007-181691 A
Patent Document 9: JP 2007-181692 A

### Summary of the Invention

#### Problems to be Solved by the Invention

However, at present, as the compound having an anticoagulant activity to be immobilized on the surface of medical devices or medical materials such as blood circuit and the like, a specific compound which can inhibit both of the blood coagulation reactions in the primary hemostasis stage in which platelets are involved and in the coagulation thrombus formation stage in which blood coagulation factors are involved has not yet been developed. Even if a conventional compound having an anticoagulant activity is to be immobilized on the surface of a medical device or medical material, it is difficult to immobilize the compound retaining a sufficient anticoagulant activity, and even when the immobilization is successful, there is a problem in that the immobilized compound is released from the medical device or the medical material into the blood. Further, in cases where a plurality of compounds are used for inhibiting both of the blood coagulation reactions in the primary hemostasis stage in which platelets are involved and in the coagulation thrombus formation stage in which blood coagulation factors are involved, it is necessary to control the competitive adsorption between the compounds and to control the immobilization ratio, which is very complicated.

Accordingly, an object of the present invention is to provide a hydrophilic polymer compound which can inhibit both of the blood coagulation reactions in the primary hemostasis stage in which platelets are involved and in the

coagulation thrombus formation stage in which blood coagulation factors are involved, which hydrophilic polymer compound can be firmly immobilized on the surface of medical devices or medical materials, in the state retaining the anticoagulant activity. Means for Solving the Problems

To solve the above-described problems, the present inventors intensively studied to discover that a hydrophilic polymer compound comprising a polymer compound which inhibits platelet adhesion and a compound which inhibits blood coagulation reaction, bound to the polymer compound, exhibits an outstanding anticoagulant activity, and can be firmly immobilized on the surface of medical devices and medical materials.

That is, the present invention provides a hydrophilic polymer compound comprising a polymer compound which inhibits platelet adhesion, and a compound that inhibits blood coagulation reaction, bound to the above-described polymer compound.

The above-described polymer compound which inhibits platelet adhesion is preferably a copolymer composed of a hydrophobic polymer and a hydrophilic polymer, and has an amount of adsorption to poly(methyl methacrylate) of not less than 0.1 pg/mm$^2$, more preferably, a copolymer of monomers selected from the group consisting of ethylene glycol, vinyl acetate, vinyl pyrrolidone, propylene glycol, vinyl alcohol and siloxane, still more preferably, a polyether-modified silicone.

The above-described compound which inhibits blood coagulation reaction preferably has an antithrombin activity, is a compound represented by General Formula (I) below, more preferably (2R,4R)-4-methyl-1-((2S)-2-{[(3RS)-3-methyl-1,2,3,4-tetrahydroquinolin-8-yl]sulfonyl}amino-5-guanidinopentanoyl)piperidin-2-carboxylic acid.

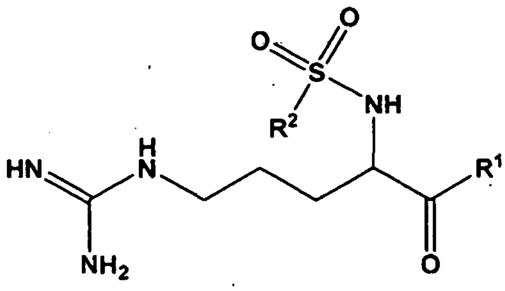
··· (I)

[wherein R$^1$ represents a (2R,4R)-4-alkyl-2-carboxypiperidino group; R$^2$ represents a phenyl group or a fused polycyclic compound residue, the fused polycyclic compound residue optionally being substituted with a lower alkyl group, a lower alkoxy group or an amino group which is substituted with a lower alkyl group].

The present invention also provides a surface treatment agent for medical devices or medical materials, the surface treatment agent comprising the above-described hydrophilic polymer compound, which has an anticoagulant activity.

The present invention further provides a medical device or a medical material treated with the above-described surface treatment agent.

## Effects of the Invention

By the present invention, both of the blood coagulation reactions in the primary hemostasis stage in which platelets are involved and in the coagulation thrombus formation stage in which blood coagulation factors are involved can be prominently inhibited, and the hydrophilic polymer compound can be firmly immobilized on the surface of medical devices or medical materials, in the state retaining the anticoagulant activity. Further, the hydrophilic polymer compound of the present invention can be used as a surface treatment agent for giving anticoagulant activity to medical devices or medical materials.

## Brief Description of the Drawings

Fig. 1 is a schematic view showing the mini-module prepared in an example.

Fig. 2 is a schematic view showing the closed circuit used in an *in vitro* blood circulation test.

Fig. 3 is a schematic view showing the human blood plasma circulation circuit used in the measurement of the amount of eluted hydrophilic polymer compound.

Mode for Carrying out the Invention

Unless otherwise specified, the terms used herein have the following definitions:

The "hydrophilic polymer compound" of the present invention is characterized in that a polymer compound which inhibits platelet adhesion and a compound which inhibits blood coagulation reaction are bound.

The term "hydrophilic" herein means that the compound is water-soluble, or even if the compound is not water-soluble, the compound interacts with water molecules by electrostatic interaction or hydrogen bond. The "hydrophilic polymer compound" includes, the hydrophilic polymer compounds wherein a copolymer of the monomers selected from the group consisting of ethylene glycol, vinyl acetate, vinyl pyrrolidone, propylene glycol, vinyl alcohol and siloxane, is bound with a compound represented by the following General Formula (I):

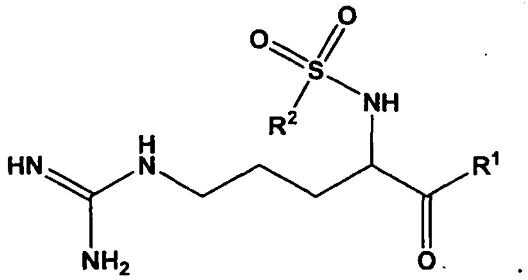

... (I)

[wherein $R^1$ represents a (2R,4R)-4-alkyl-2-carboxypiperidino group; $R^2$ represents a phenyl group or a fused polycyclic compound residue, the fused polycyclic compound residue optionally being substituted with a lower alkyl group, a lower alkoxy group or an amino group which is substituted with a lower alkyl group].

The term "polymer compound which inhibits platelet adhesion" herein means a polymer compound having a number average molecular weight of not less than 1000, which has a blood compatibility and can inhibit platelet adhesion to the surface of a substrate or material by making the polymer compound exist on the surface of a medical device or a medical material.

Examples of the "polymer compound which inhibits platelet adhesion" include poly(vinyl alcohol), poly(vinyl pyrrolidone), poly(ethylene glycol), poly(propylene glycol), polymer compounds composed of polyether and polysiloxane, polyethyleneimine, polyallylamine, polyvinylamine, poly(vinyl acetate), poly(acrylic acid), polyacrylamide, poly(hydroxyethyl methacrylate), or a copolymer or graft of monomer of these polymer compounds and other monomer, which may preferably have amino group, carboxyl group, hydroxyl group, epoxy group or mercapto group to bind with a compound which inhibits blood coagulation, more preferably a copolymer composed of a hydrophobic polymer and a hydrophilic polymer for adsorption to the surface of medical devices or medical materials, still more preferably a polymer compound composed of polyether and polysiloxane which are highly hydrophilic, or a partially-saponified poly(vinyl alcohol) or a copolymer of vinyl pyrrolidone and vinyl acetate.

Examples of the "polymer compound composed of polyether and polysiloxane" include copolymers, polymer complexes or polymer blends of polyether and polysiloxane. The copolymer of polyether and polysiloxane is composed of polyether units and polysiloxane units, and the copolymer form thereof may be any of random copolymer, block copolymer or graft copolymer. Among these, polyether-modified silicone which is highly hydrophilic is preferred.

Examples of "polyether" include poly(ethylene oxide) and structures originated from polyethylene oxide. "Polyether" herein means the structure represented by General Formula (II) ($R^3$ represents an alkyl group having not more than 6 carbon atoms), and "structure originated from polypropylene glycol" which is one example of polyether means the structure represented by General Formula (III).

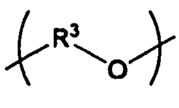

... (II)

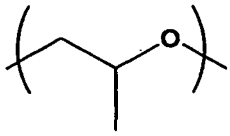

... (III)

"Polyether-modified silicone" means silicone wherein polyether units bind to side chains of silicone chains, and polyether-modified silicone which is amino-modified or carboxy-modified may also be employed.

When the polymer compound which inhibits platelet adhesion is partially-saponified poly(vinyl alcohol), the degree of saponification is preferably 50 to less than 100 mol%, more preferably 74 to 99.9 mol%, still more preferably 78 to 95 mol% from the viewpoint of attaining a preferred ease of handling or hydrophilicity. "degree of saponification" herein means a value calculated according to the Equation 1.

$$\text{Saponification level} = m/(n+m)\times 100 \text{ ......Equation 1}$$

m: the number of structures represented by General Formula (IV) in poly(vinyl alcohol)
n: the number of structures represented by General Formula (V) in poly(vinyl alcohol)

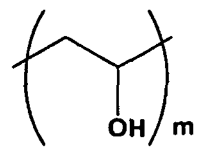
... (IV)

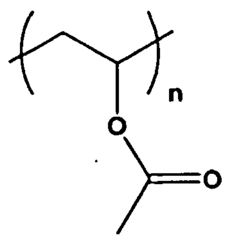
... (V)

When the polymer compound which inhibits platelet adhesion is a copolymer of vinyl pyrrolidone and vinyl acetate, vinyl pyrrolidone units are preferably not less than 50 unit mol%, more preferably not less than 60 unit mol%, from the viewpoint of attaining a preferred ease of handling or hydrophilicity. On the other hand, from the viewpoint of attaining a preferred amount of adsorption to base material, vinyl pyrrolidone units are preferably less than 100 unit mol%. The percentage of vinyl pyrrolidone units in a copolymer of vinyl pyrrolidone and vinyl acetate (unit mol%) is calculated by $^1$H-NMR measurement (solvent: $CDCl_3$) of the copolymer.

The amount of adsorption of the polymer compound which inhibits platelet adhesion, to base materials such as medical device, medical material or the like, is preferably not less than 0.1 pg/mm$^2$, more preferably not less than 1 pg/mm$^2$, still more preferably not less than 10 pg/mm$^2$.

The amount of adsorption is measured by the following method: Firstly, untreated sensor chip (Sensor Chip Au; GE Healthcare) is pretreated (distilled water at 25°C, at a flow rate of 20 μl/min, for 10 minutes) using a surface plasmon resonance system (hereinafter referred to as "SPR") (BIACORE 3000; GE Healthcare), and the signal value (RU: resonance unit) is measured.

"Base material", i.e., an adsorbent material is dissolved into a solvent to prepare a 0.5wt% solution of the adsorbent material. One drop of the solution of the adsorbent material is dropped on to the center of the gold film part and the resultant is immediately rotated at 3000 rpm for 1 minute at room temperature to coat the sensor chip with the adsorbent material.

After confirming that no droplet exists on the sensor chip, the sensor chip is washed with distilled water using SPR (at 25°C, at a flow rate of 20 μl/min, for 10 minutes), and further washed three times with 0.025wt% Triton-X100 solution (at 25°C, at a flow rate of 20 μl/min, for 1 minute), and then the signal value at 10 minutes after the end of washing is measured.

Among the sensor chips obtained by the method described above, the one whose signal value difference before and after spin coat was within the range from 3000 to 8000 was selected, then washed with distilled water (at 25°C, at a flow rate of 20 μl/min, for 10 minutes), and further washed three times with 0.025wt% Triton-X100 solution (at 25°C, at a flow rate of 20 μl/min, for 1 minute).

Ten minutes after the end of washing, an aqueous solution of a hydrophilic polymer compound to be adsorbed to the base material (concentration: 100 μg/ml) is injected (at 25°C, at a flow rate of 20 μl/min, for 1 minute), and then the resultant is washed with distilled water (at 25°C, at a flow rate of 20 μl/min, for 3 minute). The difference between

the signal value immediately before injection (hereinafter referred to as "signal value A") and the signal value at 3 minutes after the end of the injection (hereinafter referred to as "signal value B") is determined, which is then converted as 1 RU = 1 $pg/mm^2$.

Next, the sensor chip is washed with distilled water (at 25°C, at a flow rate of 20 μl/min, for 2 minutes), and further washed three times with 0.025wt% Triton-X100 solution (at 25°C, at a flow rate of 20 μl/min, for 1 minute), and again the aqueous solution of the hydrophilic polymer compound to be adsorbed (concentration: 100 μg/ml) is injected (at 25°C, at a flow rate of 20 μl/min, for 1 minute). Thereafter, the same steps are repeated to determine signal differences 5 times in total, and the mean value is regarded as "the amount of adsorption of polymer compound which inhibits platelet adhesion to base materials".

"Compound which inhibits blood coagulation reaction" means a compound having an anti-coagulation activity such as an antithrombin activity, and more specifically means a compound which prolongs the prothrombin time than native blood by not less than 30% when the compound is added into blood to a concentration of 10 μg/mL.

"Prothrombin time" is measured by the method according to a known literature (Masamitsu Kanai et al., "Clinical Test Handbook, vol. 30", Kanihara shuppan, 1993, p416-418). More specifically, 1 part by volume of 3.2% sodium citrate and 9 parts by volume of blood are mixed, and 0.1 mL aliquot of the sodium citrate-blood plasma is sampled into a small test tube (inner diameter: 8mm, length: 7.5cm), followed by heating the resultant in a thermostat bath at 37°C for 3 minutes. Then 0.2 mL of tissue thromboplastin-calcium reagent kept at 37°C is added thereto, and then the small test tube is lightly shaken, followed by leaving the small test tube to stand to precipitate fibrin. Here, the time required for the precipitation of fibrin after addition of the tissue thromboplastin-calcium reagent is measured, which measured time is defined as "prothrombin time".

Examples of the "compound which inhibits blood coagulation reaction" include heparin, nafamostat mesilate, sodium citrate, sodium oxalate, α1-antitrypsin, α2-macroglobulin, C1 inhibitor, thrombomodulin, protein C, compounds having guanidine structure, prostaglandins, hirudin, Xa inhibitors, tissue factor inhibitor and antithrombin, and preferred are compounds having an antithrombin activity.

"Compound having antithrombin activity" means a compound which has a high binding affinity to thrombin.

An example of the index to evaluate the antithrombin activity of compounds is the inhibition constant (hereinafter referred to as "Ki") which is calculated from Lineweaver-Burk plot based on absorbance value of a test solution. Lower Ki indicates higher binding affinity to thrombin and higher antithrombin activity.

Examples of the "compound having antithrombin activity" include compounds having guanidine structure, and preferred is (2R,4R)-4-methyl-1-((2S)-2-{[(3RS)-3-methyl-1,2,3,4-tetrahydroquinolin-8-yl]sulfonyl}amino-5-guanidinopentanoyl)piperidin-2-carboxylic acid(hereinafter referred to as "argatroban"). Argatroban, which was synthesized in 1978, is a medicinal compound having a selective antithrombin activity of an arginine derivative.

The surface treatment agent of medical device or medical material of the present invention is characterized by containing the above-described hydrophilic polymer compound and having an anticoagulant activity.

Examples of the "medical device or medical material" include implantable artificial organs, artificial blood vessels, catheters, stents, blood bags, contact lenses, intraocular lenses and surgery technical aids; and separation membranes and adsorbents which are included in modules for biogenic substance separation, hemocatharsis or the like.

Methods for treating the surface of the medical devices or medical materials using the above-described surface treatment agent, i.e., methods for immobilizing the above-described hydrophilic polymer compound which is active ingredient thereof on the surface of the medical devices or medical materials include, for example, a method wherein the above-described surface treatment agent is made to contact the medical devices or medical materials and then irradiated with a radiation. As the type of radiation, electron beam or γ-ray is preferred.

Examples of the material constituting the "medical devices or medical materials" include cellulose, cellulose acetate, polycarbonates, polysulfones, poly(ether sulfones), polymethacrylates such as poly(methyl methacrylate) (hereinafter referred to as "PMMA"), polyacrylates, polyamides, poly(vinylidene fluoride), poly(vinyl chloride), polyacrylonitrile, polyesters, polyurethanes, polystyrene, polyethylene, polypropylene, poly(vinylidene fluoride), polymethylpentene and polyimides.

## Examples

The present invention will now be described in more detail referring to examples. However, the present invention is not restricted thereto.

(Example 1: Binding between Amino-polyether-modified Silicone and Argatroban)

Argatroban in an amount of 5 mmol was placed in a recovery flask, and 10 mL of anhydrous dimethylformamide (hereinafter referred to as "anhydrous DMF") was added thereto to dissolve the argatroban. Thereafter, 10 mL of 4N hydrochloric acid/1,4-dioxane (TOYOKASEI) was added dropwise while cooling the recovery flask and the resulting

mixture was stirred for 1 hour. Then the solvent was evaporated with a rotary evaporator and the resultant was further dried overnight in a vacuum dryer, followed by addition of 25 mL of anhydrous DMF to obtain argatroban hydrochloric acid salt solution in anhydrous DMF.

Argatroban hydrochloric acid salt solution in anhydrous DMF in an amount shown in Table 1 was placed in a two-necked flask, and dicyclohexylcarbodiimide (hereinafter referred to as "DCC") solution in anhydrous DMF and 4-hydroxybenzotriazole (hereinafter referred to as " HOBt") solution in anhydrous DMF were added. Then polyether-modified silicone (X-22-3939A (SHIN-ETSU CHEMICAL) was further added and the resulting mixture was allowed to react at room temperature for 3 days. Then the reaction solution was placed in a dialysis tube (SPECTRAPORE RC, PORE 6, MWCO=1000), and dialyzed for 3 days against distilled water with a volume of more than 10 times while appropriately replacing the distilled water. The reaction solution after the dialysis was filtered, and the solvent in the filtrate was evaporated with a rotary evaporator, followed by drying the resultant overnight in a vacuum dryer to obtain a hydrophilic polymer compound (hereinafter referred to as "Example 1 Compound").

(Measurement of Antithrombin Activity of Example 1 Compound)

ECA-T kit (HAEMOSYS) was used for the measurement. To 100 μL of Example 1 Compound, 900 μL of distilled water was added to prepare an aqueous Example 1 Compound solution. The Example 1 Compound solution in an amount of 30 μL was sampled, and mixed with 100 μL of ECA prothrombin buffer and 25 μL of ECA-T substrate. After incubating the resulting mixture at 37°C for 60 seconds, the mixture was set in an apparatus (COATRON M1 (code 80 800 000); PRODUCTION) and measurement was carried out after adding 50 μL of ECA ecarin reagent.

A mixture of 20 μL of argatroban solution prepared to an arbitrary concentration using ethanol/hydrochloric acid (volume ratio: 4/1) mixed solvent and 80 μL of human blood plasma, and a mixture of 20 μL of blank distilled water and 80 μL of human blood plasma were subjected to the measurement, respectively, in place of the above-described aqueous Example 1 Compound solution, using the ECA-T kit, and a calibration curve was prepared from the results thereof. The concentration of 1494.3 ppm by weight in terms of argatroban of the aqueous Example 1 Compound solution calculated from the calibration curve was defined as the value indicating the antithrombin activity of Example 1 Compound.

(Examples 2 to 13)

Examples 2 to 13 Compounds were obtained, respectively, in the same manner as in Example 1 except that the molar ratios of DCC, HOBt and polyether-modified silicone (X-22-3939A) to argatroban hydrochloric acid salt and the volume ratio of anhydrous DMF to polyether-modified silicone were changed to measure the antithrombin activity thereof. The molar ratios of DCC, HOBt and polyether-modified silicone (X-22-3939A) to argatroban and the measurement results of the antithrombin activity of each Examples 2 to 13 Compounds are shown in Table 1.

Table 1

| Compounds | Molar ratios per 1.00 mole of argatroban hydrochloric acid salt | | | Volume ratios of anhydrous DMF per 1 volume of polyether-modified silicone | Concentration in terms of argatroban (ppm by weight) |
|---|---|---|---|---|---|
| | DCC | HOBt | X-22-3939A | | |
| Example 1 | 1.07 | 1.06 | 0.060 | - | 1494.3 |
| Example 2 | 1.04 | 1.04 | 0.060 | - | 831.2 |
| Example 3 | 0.20 | 0.20 | 0.060 | 1.4 | 6610.7 |
| Example 4 | 0.20 | 0.20 | 0.030 | 3.9 | 8393.3 |
| Example 5 | 1.29 | 1.27 | 0.493 | 1.8 | 505.3 |
| Example 6 | 1.29 | 1.27 | 0.203 | 4.3 | 771.7 |
| Example 7 | 1.29 | 1.27 | 0.101 | 8.6 | 606.7 |
| Example 8 | 1.29 | 1.27 | 0.067 | 13.0 | 441.7 |
| Example 9 | 1.29 | 1.27 | 0.049 | 17.6 | 436.7 |
| Example 10 | 1.29 | 1.27 | 0.020 | 42.9 | 738.9 |
| Example 11 | 1.29 | 1.27 | 0.010 | 88.2 | 895.0 |
| Example 12 | 1.00 | 1.00 | 0.060 | - | 6000.0 |

(continued)

| Compounds | Molar ratios per 1.00 mole of argatroban hydrochloric acid salt | | | Volume ratios of anhydrous DMF per 1 volume of polyether-modified silicone | Concentration in terms of argatroban (ppm by weight) |
|---|---|---|---|---|---|
| | DCC | HOBt | X-22-3939A | | |
| Example 13 | 1.00 | 1.00 | 0.060 | 40.0 | 5999.4 |

The antithrombin activity of a polyether-modified silicone (X-22-3939A) was also measured, but the measured value was the same as that of the blank distilled water, and it was confirmed that the polyether-modified silicone itself does not have an antithrombin activity.

(Measurement of Thrombin Inhibition Constant of Example 1 Compound)

Aqueous thrombin from bovine plasma solution was prepared by dissolving 10,000 U of thrombin from bovine plasma solution (ITO LIFE SCIENCES) in 1 mL of physiological saline solution.

Aqueous S-2238 stock solution was prepared by dissolving 25 mg of S-2238 stock solution (SEKISUI MEDICAL) in 40 mL of distilled water.

The aqueous bovine thrombin solution, aqueous S-2238 stock solution and the above-described aqueous Example 1 Compound solution were each diluted by using diluted buffer solution (0.05M Tris, 0.1M NaCl, 1 mg/mL of bovine serum albumin (BSA), pH 7.4).

To a 96-well plate, 100 μL of diluted solution of the aqueous S-2238 stock solution and 50 μL of diluted solution of the aqueous Example 1 Compound solution were dispensed, and the resultant was sealed and then warmed in a thermostat dryer set at 37°C for 30 minutes. Thereafter, 50 μL of the diluted solution of the aqueous thrombin from bovine plasma solution which was heated at 37°C for 30 minutes was further dispensed thereto, and absorbance of the resulting mixture was immediately measured with a microplate reader (measurement wavelength: 405 nm, reference wavelength: 595nm).

Immediately after finishing the first measurement of the absorbance, the second measurement of the absorbance was carried out. The third or later measurements of the absorbance were carried out at 4, 6, 8, 10, 12, 14, 16, 18 and 20 minutes, respectively, after the diluted solution of aqueous thrombin from bovine plasma solution was dispensed. Ki was calculated from each value of the obtained absorbances by using Lineweaver-Burk plot. The Ki of the Example 1 Compound was 21 nM.

The Ki of the polyether-modified silicone (X-22-3939A) was also calculated in the same manner, but the Ki of the polyether-modified silicone without an antithrombin activity was the same as that of the blank, as expected.

Further, the Ki of argatroban was also calculated in the same manner, and the Ki was 42 nM which was not less than twice the value of the Ki of the Example 1 Compound.

From these results, it is apparent that the above-described hydrophilic polymer compound has an extremely high binding affinity to thrombin, and can give a prominent antithrombin activity much higher than the argatroban which is known to have an antithrombin activity, to medical devices including hollow fiber dialyzer or medical material.

(Preparation of PMMA Hollow Fiber Membrane Mini-module)

Five parts by weight of isotactic-PMMA and 20 parts by weight of syndiotactic-PMMA were added to 75 parts by weight of dimethylsulfoxide, and the resulting mixture was stirred at 110°C for 8 hours to obtain a membrane-forming liquid. The obtained membrane-forming liquid was extruded from an orifice type coaxial cylindrical mouthpiece, and after passing 300 mm in the air, the extruded material was introduced into a coagulation bath containing 100% water, thereby PMMA hollow fibers having an inner diameter of 0.2 mm and a thickness of a membrane of 0.03 mm were obtained. As the gas introduced into the inside of the fiber, dry nitrogen was used.

A module case having an inner diameter of 10 mm and a length of 120 mm, which had two ports communicating to the inside of the hollow fibers (blood port) and two ports communicating to the outside of the hollow fibers (dialysate port), respectively, was prepared.

Fifty of the above-described PMMA hollow fibers were bundled to form PMMA hollow fiber membranes, and both ends of the PMMA hollow fiber membranes were fixed to the above-described module case by using an epoxy potting material with attention not to clog the hollow portions of the PMMA hollow fiber membranes. Thereafter, the PMMA hollow fiber membranes and the inner side of the module case were washed with distilled water to obtain Mini-module 6 as shown in Fig. 1.

(Immobilization of Example 1 Compound to PMMA Hollow Fiber Membranes)

Bis-Tris (DOJINDO LABORATORIES) and sodium chloride were dissolved in ultrapure water so as to attain a final concentration of 0.25M and 0.5M, respectively, and pH of the resulting solution was adjusted to 5 by adding 6N hydrochloric acid dropwise to prepare Bis-Tris buffer solution having 5 times concentration.

Distilled water remained at the side contacting the blood (the inner side of the PMMA hollow fiber membranes) and the side not contacting the blood (the outer side of the PMMA hollow fiber membranes) of the prepared Mini-module 6 was removed with compressed air. Then aqueous Example 1 Compound solution in a concentration of 4000 ppm by weight in terms of argatroban, propylene glycol and Bis-Tris buffer solution having 5 times concentration were mixed in a volume ratio of 5/3/2 to obtain a filling solution.

Only at the side contacting the blood of the Mini-module 6, the above-described filling solution(400 μL) was filled using a syringe. Thereafter, the filling solution was removed with compressed air, and all of the blood ports 1a and 1b and the dialysate ports 2a and 2b were tightly capped to irradiate the Mini-module 6 with γ-ray at a dose of 25 kGy for 3 hours.

The PMMA hollow fiber membranes 4 and the inner side of the Mini-module 6 were washed by flowing 0.025% by weight of aqueous poly(oxyethylene octyl phenyl ether) solution into the PMMA hollow fiber membranes 4 and the inner side of the Mini-module 6 at a flow rate of 10 mL/min for 8 hours by using a peristaltic pump 8. Thereafter, distilled water and physiological saline solution were flown at a flow rate of 10 mL/min for 30 minutes, respectively, to carry out further wash to obtain a mini-module in which the Example 1 Compound was immobilized (hereinafter referred to as "Example 1 Mini-module").

A mini-module in which polyether-modified silicone was immobilized (hereinafter referred to as "Comparative Example 1 Mini-module") was obtained by carrying out the same procedure described above except that polyether-modified silicone (X-22-3939A) was used in place of aqueous Example 1 Compound solution in a concentration of 4000 ppm by weight in terms of argatroban.

(*In vitro* Test of Blood Circulation)

The blood supplied by a volunteer and citric acid was mixed in a volume ratio of 9/1 to obtain blood supplemented with citric acid. Calcicol in an amount of 43.6 μL was added to 1 mL of the blood supplemented with citric acid to obtain a test blood.

Silicone tubes 7a and 7b were connected to the Example 1 Mini-module, and a peristaltic pump 8 was placed in the middle of the silicone tube 7b. The test blood was flown at a flow rate of 0.9 mL/min for 5 seconds from silicone tube 7a connected to the blood port 1a, and the test blood discharged from the blood port 1b was discarded from silicone tube 7b to remove bubbles in the inner side of the PMMA hollow fiber membranes. Then, the silicone tubes 7a and 7b were connected at Connecting part 9 to form a closed circuit shown in Fig.2.

The circulation of the test blood was started at a flow rate of 0.9 mL/min to measure the duration of circulation until the silicone tubes 7a or 7b came off the Connecting part 9 due to an increased inner pressure in the circuit caused by coagulation thrombus formed in the circuit. The duration of circulation when using the Example 1-Mini-module was 46 minutes.

Mini-module 6 in which no compound was immobilized on the PMMA hollow fiber membranes (hereinafter referred to as "Comparative Example 2 Mini-module") was prepared to carry out the same test of blood circulation as described above. The duration of circulation in this case was 20 minutes which was not more than half the duration of circulation when using Example 1 Mini-module. From these results, it is apparent that the above-described hydrophilic polymer compounds can give a prominent anticoagulant activity to medical devices including hollow fiber dialyzer or medical materials.

When the same test of blood circulation was carried out as described above by using Comparative Example 1 Mini-module, the duration of circulation was 20 minutes, which was the same as the duration of circulation when using Comparative Example 2 Mini-module in which no compound was immobilized on the PMMA hollow fiber membranes.

(Measurement of Eluted amount of Example 1 Compound)

Silicone tube 7b having an inner diameter of 0.8 mm and a length of 520 mm was connected to the blood port 1b of the separately prepared Example 1 Mini-module, and peristaltic pump 8 was placed in the middle of the silicone tube 7b. Silicone tube 7a having an inner diameter of 0.8 mm and a length of 160 mm was connected to the blood port 1a. Thereafter, other ends of the silicone tubes 7a and 7b were each inserted in polystyrene round tube (Code: 352054; BECTON DICKINSON) 10 containing 5 mL of human plasma to prepare circulating circuit shown in Fig. 3.

After circulation in human plasma at a flow rate of 0.5 mL/min for 4 hours by using peristaltic pump 8, the concentration of the Example 1 Compound in human plasma in polystyrene round tube 10 was measured by using ECA-T kit. However, the concentration of the Example 1 Compound in human plasma after circulation was below the detection limit of ECA-T kit, and elution of the Example 1 Compound from Example 1 Mini-module was not confirmed. This result shows that the above-described hydrophilic polymer compound can be immobilized strongly to medical devices including hollow fiber dialyzer or medical materials.

(Evaluation of Amount of Adsorption of Polymer Compounds having Platelet Adhesion Inhibitory Activity)

PVP (K-90), VA73, VA64, VA55 and VA37 (any of these were from BASF Corporation) were provided as a copolymer between vinylpyrrolidone and vinyl acetate (hereinafter referred to as "VA Copolymer"), which copolymer constitutes the above-described hydrophilic polymer compounds and was one of polymer compounds inhibiting platelet adhesion. Similarly, PVA217, PVA417 and PVA205c (any of these were from KURARAY) were provided as a partially saponified poly(vinyl alcohol) which was one of the polymer compounds inhibiting platelet adhesion. Furthermore, as a polyether-modified silicone, F114, F244, F303, F3031, F348, F350s, F502, F506 and X-22-3939A (any of these were from SHIN-ETSU SILICONE) were provided. The prepared VA Copolymer, partially saponified poly(vinyl alcohol) and polyether-modified silicone were each diluted with distilled water to prepare 10,000 ppm by weight of aqueous solution.

On the other hand, as polymer compounds constituting the above-described hydrophilic polymer compounds, which were not included in the polymer compounds inhibiting platelet adhesion, PEG2000, PEG4000, PEG6000 and PEG20000 (any of these were from NACALAI TESQUE), and PEG-methyl ether (PEG-em) and PEG-dimethyl ether (PEG-dm) (both were from SIGMA-ALDRICH) were provided for comparison. The prepared polymer compounds were each diluted with distilled water to prepare 10,000 ppm by weight of aqueous solution.

As 0.5% by weight solutions of adsorbent materials adsorbing polymer compounds which inhibit platelet adhesion, PMMA (weight average molecular weight: 93000, SIGMA-ALDRICH) solution in toluene, polyurethane solution in dimethylacetamide, polysulfone (Udel (registered trademark) P-3500 produced by SOLVAY) solution in dimethylacetamide, poly(vinyl chloride) (weight average molecular weight: 80000, SIGMA-ALDRICH) solution in tetrahydrofuran, polystyrene (WAKO) solution in chloroform and polycarbonate (weight average molecular weight: 20000, TEIJIN) solution in chloroform were each prepared.

The amounts of adsorption of various polymer compounds inhibiting platelet adhesion were measured for each adsorbent material. The results are shown in Table 2.

Table 2

| Signal value B - Signal value A [pg/mm²] | | | | | | | |
|---|---|---|---|---|---|---|---|
| | | Adsorbent materials | | | | | |
| | | PMMA | Poly-sulfone | Poly-urethane | Poly(vinyl chloride) | Poly-styrene | Poly-carbonate |
| [illegible] | PVPK90 | 789 | - | - | - | - | - |

| | | | | | | | |
|---|---|---|---|---|---|---|---|
| | VA37 | 2760 | - | - | - | - | - |
| | VA55 | 472 | - | - | - | - | - |
| | VA64 | 920 | - | - | - | - | - |
| | VA73 | 426 | - | - | - | - | - |
| | PVA 217 | 2529 | 2886 | 1635 | 2468 | 2777 | 2356 |
| | PVA417 | 2475 | 2742 | 1911 | 2330 | 2662 | 2346 |
| | PVA205c | 2223 | 2130 | 1411 | 1796 | 1989 | 1819 |
| | F114 | 1003 | 844 | 514 | 739 | 621 | 756 |
| | F244 | 1639 | 1272 | 1144 | 1118 | 1052 | 1243 |
| | F303 | 1268 | 1156 | 1604 | 1037 | - | 1374 |
| | F3031 | 947 | 559 | 614 | 418 | 339 | 536 |
| | F348 | 875 | 784 | 756 | 608 | 283 | 800 |
| | F350s | 751 | 657 | 674 | 544 | 275 | 591 |
| | F502 | 827 | 657 | 696 | 385 | 197 | 482 |
| | F506 | 691 | 308 | 437 | 167 | 43 | 279 |
| | X-22-3939A | 1182 | 910 | 1204 | 695 | 924 | 1424 |
| | PEG2000 | 2 | - | - | - | - | - |
| | PEG4000 | 2 | - | - | - | - | - |
| | PEG6000 | 5 | - | - | - | - | - |
| | PEG20000 | 113 | - | - | - | - | - |
| | PEG-me | 5 | - | - | - | - | - |
| | PEG-dm | 67 | - | - | - | - | - |

From the results in Table 2, it is apparent that the polymer compounds constituting the above-described hydrophilic polymer compounds, which inhibit platelet adhesion, are not limited to polyether-modified silicone (X-22-3939A), and can be adsorbed strongly to medical devices including hollow fiber dialyzer or medical materials.

(Evaluation of Platelet Adhesion Inhibitory Activity)

The separately prepared module case of Example 1 Mini-module was cut with ultrasonic disc cutter to take out the PMMA hollow fiber membranes (hereinafter referred to as "Example 1 Hollow Fiber Membranes") on which Example 1 Compound was immobilized.

A double-stick tape was adhered to one surface of a poly(ethylene terephthalate) circular film having a diameter of 18 mm, and after fixing the Example 1 Hollow Fiber Membranes thereto, the fixed PMMA hollow fiber membranes were cut into semicylindrical shape to expose the inner surfaces of the PMMA hollow fiber membranes. The Example Hollow Fiber Membranes fixed to the circular film was attached to a Falcon (registered trademark) cylindrical tube cut into cylindrical shape (diameter 18 mm, NO. 2051), and the gap between the cylindrical tube and the circular film was sealed with Parafilm. Thereafter, the cylindrical tube was filled with physiological saline solution.

Venous blood right after collection from a volunteer was placed in a blood collection tube in which heparin was preliminarily collected, and the resulting mixture was mixed by upside-down mixing to prepare blood supplemented with heparin. The concentration of the blood supplemented with heparin was set to 50 U/mL.

After discarding the physiological saline solution in the above-described cylindrical tube, 1.0 mL of the blood supplemented with heparin was added thereto, and the cylindrical tube was shaken at 37°C for 1 hour. Thereafter, Example 1 Hollow Fiber Membranes in the above-described cylindrical tube was washed with 10 mL of physiological saline solution, and then blood components were fixed by adding physiological saline solution containing 2.5% by volume

of glutaraldehyde, followed by further washing the membranes with distilled water. Then, the circular film fixing Example 1 Hollow Fiber Membranes was removed from the above-described cylindrical tube, and the circular film fixing Example Hollow Fiber Membranes was dried under reduced pressure at normal temperature at an absolute pressure of 0.5 Torr for 12 hours.

The circular film dried under reduced pressure on which Example 1 Hollow Fiber Membranes was fixed was adhered to the stage of a scanning electron microscope with a double-stick tape, and platinum/palladium thin film was then formed on the surface of Example Hollow Fiber Membranes by sputtering. The inner surfaces in the center portion in the longitudinal direction of the Example Hollow Fiber Membranes on which surface platinum/palladium thin film was formed were observed with a field emission scanning electron microscope (S800 produced by HITACHI) at a magnification of x 1500, and the number of adhered platelets in one visual field (4.3 x $10^3$ $\mu$m2) was counted.

The integer value of the mean of the numbers of adhered platelets counted in different 5 visual fields is defined as the number of adhered platelets (platelets/(4.3 x $10^3$ $\mu m^2$)), and the number of adhered platelets to Example 1 Hollow Fiber Membranes was one.

On the other hand, the separately prepared module case of Comparative Example 2 Mini-module was cut with ultrasonic disc cutter, and hollow fiber membranes in which any compound was not immobilized (hereinafter referred to as "Comparative Example 2 Hollow Fiber Membranes") were taken out to confirm the number of adhered platelets as well. As a result, the number of adhered platelets of Comparative Example 2 Hollow Fiber Membranes was not less than 100.

From these results, it is apparent that the above-described hydrophilic polymer compounds can give a prominent platelet adhesion inhibitory activity to medical devices including hollow fiber dialyzer or medical materials.

(Measurement of Whole Blood Clotting Time)

The blood collected from a volunteer and citric acid were mixed at a volume ratio of 9/1 to prepare blood supplemented with citric acid.

In a cuvette (NON-ACTIVATED CLOTTING TEST KIT), 18 $\mu$L of physiological saline solution was placed, and 14.8 $\mu$L of calcicol was added thereto, followed by further adding 342 $\mu$L of blood supplemented with citric acid. Thereafter, the measurement using Sonoclot Blood Coagulation/Platelet Function Analyzer (IMI) was carried out to define the obtained ACT ONSET value as whole blood clotting time. The whole blood clotting time of the blood collected by volunteer was 545 seconds.

The whole blood clotting times were 531, 746 and 849 seconds, respectively, when the same measurements were carried out using 2, 10 and 20 $\mu$M of argatroban solutions (solvents: methanol/hydrochloric acid (volume ratio: 4/1)), respectively, in place of physiological saline solution.

The whole blood clotting times were 527, 693 and 730 seconds, respectively, when the same measurements were carried out using 0.3, 1.3 and 2.5 $\mu$M of aqueous Example 1 Compound solutions, respectively, in place of physiological saline solution.

(Example 14: Binding between Vinyl acetate-Vinylpyrrolidone Copolymer and Argatroban)

In a screw vial, 14.9 g of tetrahydrofuran, 11.5 g of vinyl acetate, 10.8 g of N-vinylpyrrolidone, 0.028 g of 2-aminoethanethiol and 0.016 g of azobisisobutyronitrile were placed, and after sealing the screw vial, the resulting mixture was sonicated for 10 minutes. The seal of the screw vial was once taken off, and the mixture was bubbled with argon gas for 10 minutes, and after sealing the screw vial again, the screw vial was immersed in a hot water bath at 60°C under stirring for 1 hour, and further in a hot water bath at 70°C for 6 hours to copolymerize vinyl acetate with vinylpyrrolidone. To the obtained reaction solution, 80 mL of methanol was added, and the resulting mixture was added to about 5 times amount of ether, followed by removing the supernatant. After repeating the washing operation 3 times in which ether was newly added and the supernatant was removed, the resultant was dried under reduced pressure to obtain vinyl acetate-vinylpyrrolidone copolymer. The obtained vinyl acetate-vinylpyrrolidone copolymer was measured by $^1$H-NMR (solvent: $CDCL_3$) to obtain 60.6 unit % by mole for vinylpyrrolidone unit.

A vinyl acetate-vinylpyrrolidone copolymer solution in anhydrous DMF was prepared by dissolving 3.58 g of the obtained vinyl acetate-vinylpyrrolidone copolymer in 20 mL of anhydrous DMF. The total weight of the prepared vinyl acetate-vinylpyrrolidone copolymer solution in anhydrous DMF and 0.5 mL of argatroban hydrochloride solution in anhydrous DMF (0.49 M) were placed in a two-necked flask, and 0.5 mL of DCC solution in anhydrous DMF (1.04 M) and 0.5 mL of HOBt solution in anhydrous DMF (1.02 M) were added thereto, respectively, with ice cooling and stirring, followed by reaction under a nitrogen atmosphere at room temperature for 3 days. Then, the reaction solution was placed in a dialysis tube (SPECTRAPORE RC, PORE 6, MWCO=1000), and dialyzed for 3 days against distilled water with a volume of more than 10 times while appropriately replacing the distilled water. The reaction solution after the dialysis was filtrated, and the solvent in the filtrate was evaporated by a rotary evaporator, followed by drying the resultant

overnight in a vacuum drier to obtain a hydrophilic polymer compound (hereinafter referred to as "Example 14 Compound").

(Measurement of Antithrombin Activity of Example 14 Compound)

The antithrombin activity of Example 14 Compound solution in methanol (concentration: 20% by weight) was measured in the same manner as the measurement of the antithrombin activity of Example 1 Compound, and the calculated concentration of 104.1 ppm in terms of argatroban of the Example 14 Compound solution in methanol was defined as the value indicating the antithrombin activity of the Example 14 Compound solution in methanol.

From these results, it is apparent that the above-described hydrophilic polymer compounds can prolong the whole blood clotting time when compared with argatroban which is known to have an antithrombin activity, even if the concentrations of the hydrophilic polymer compounds are very low, and the hydrophilic polymer compounds can give an excellent anticoagulant activity to medical devices including hollow fiber dialyzer or medical materials.

(Industrial Applicability)

The present invention can be used to give an excellent anticoagulant activity to medical devices including hollow fiber dialyzer or medical materials.

(Description of Symbols)

| | |
|---|---|
| 1a, 1b | Blood port |
| 2a, 2b | Dialysate port |
| 3 | Module case |
| 4 | PMMA hollow fiber membrane |
| 5 | Potting material |
| 6 | Mini-module |
| 7a, 7b | Silicone tube |
| 8 | Peristaltic pump |
| 9 | Connecting part |
| 10 | Polystyrene round tube |

## Claims

1. A hydrophilic polymer compound comprising a polymer compound which inhibits platelet adhesion, and a compound that inhibits blood coagulation reaction, bound to said polymer compound, wherein said polymer compound which inhibits platelet adhesion is a copolymer of monomers selected from the group consisting of ethylene glycol, vinyl acetate, vinyl pyrrolidone, propylene glycol, vinyl alcohol and siloxane, and said compound that inhibits blood coagulation reaction is a compound represented by General Formula (I):

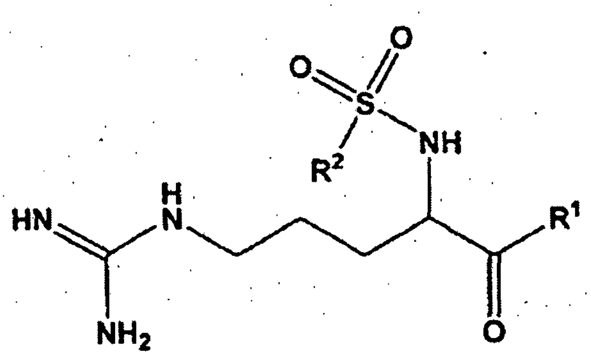

... (I)

wherein $R^1$ represents a (2R,4R)-4-alkyl-2-carboxypiperidino group; $R^2$ represents a phenyl group or a fused polycyclic compound residue, the fused polycyclic compound residue optionally being substituted with a lower alkyl group, a lower alkoxy group or an amino group which is substituted with a lower alkyl group.

2. The hydrophilic polymer compound according to claim 1, wherein said polymer compound which inhibits platelet adhesion is a copolymer composed of a hydrophobic macromolecule and a hydrophilic macromolecule, and has an amount of adsorption to poly(methyl methacrylate) of not less than 0.1 pg/mm$^2$.

3. The hydrophilic polymer compound according to claim 1 or 2, wherein said compound which inhibits blood coagulation reaction is a compound having antithrombin activity.

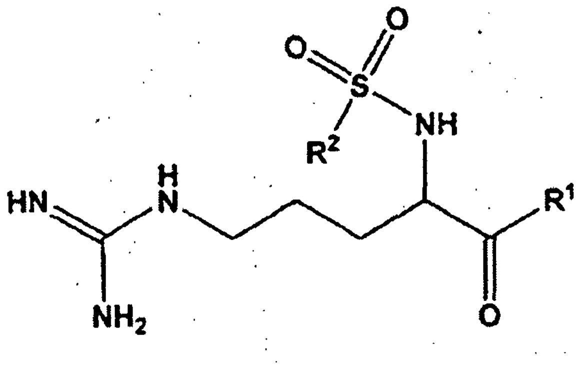

... (I)

4. The hydrophilic polymer compound according to any one of the preceeding claims, wherein said copolymer is a polyether-modified silicone.

5. The hydrophilic polymer compound according to claim 1 or 4, wherein said compound of General Formula (I) is (2R,4R)-4-methyl-1-((2S)-2-[(3RS)-3-methyl-1,2,3,4-tetrahydroquinoline-8-yl]sulfonyl]amino-5-guanidinopentanoyl)-piperidine-2-carboxylic acid.

6. A surface treatment agent for medical devices or medical materials, the surface treatment agent comprising said hydrophilic polymer compound according to any one of claims 1 to 5, which has an anticoagulant activity.

7. A medical device or a medical material treated with said surface treatment agent according to claim 6.

**Patentansprüche**

1. Hydrophile Polymerverbindung, umfassend eine Polymerverbindung, welche Thrombozytenadhäsion inhibiert und eine Verbindung, die an die Polymerverbindung gebunden ist, die eine Blutgerinnungsreaktion inhibiert, wobei die Polymerverbindung, welche die Thrombozytenadhäsion inhibiert, ein Copolymer aus Monomeren, ausgewählt aus der Gruppe bestehend aus Ethylenglykol, Vinylacetat, Vinylpyrrolidon, Propylenglykol, Vinylalkohol und Siloxan, ist und die Verbindung, die die Blutgerinnungsreaktion inhibiert, eine Verbindung ist, die durch die Allgemeine Formel (I) dargestellt wird:

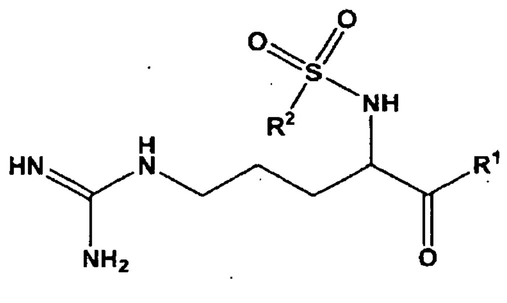

... (I)

wobei R$^1$ eine (2R,4R)-4-Alkyl-2-carboxypiperidin Gruppe repräsentiert; R$^2$ eine Phenylgruppe oder einen kondensierten polyzyklischen Verbindungsrest repräsentiert, wobei der kondensierte polyzyklische Verbindungsrest optional mit einer Niederalkylgruppe, einer Niederalkoxygruppe oder einer Aminogruppe, welche mit einer Niederalkylgruppe substituiert ist, substituiert ist.

**2.** Hydrophile Polymerverbindung gemäß Anspruch 1, wobei die Polymerverbindung, welche die Thrombozytenadhäsion inhibiert, ein Copolymer ist, welches aus einem hydrophoben Makromolekül und einem hydrophilen Makromolekül besteht und eine Adsorptionsmenge an Poly(methylmethacrylat) von nicht weniger als 0,1 pg/mm$^2$ hat.

**3.** Hydrophile Polymerverbindung gemäß Anspruch 1 oder 2, wobei die Verbindung, welche die Blutgerinnungsreaktion inhibiert, eine Verbindung ist, die Antithrombinaktivität besitzt.

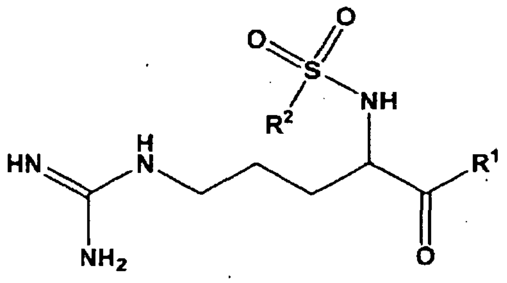

··· (I)

**4.** Hydrophile Polymerverbindung gemäß einem der vorhergehenden Ansprüche, wobei das Copolymer ein polyethermodifiziertes Silikon ist.

**5.** Hydrophile Polymerverbindung gemäß Anspruch 1 oder 4, wobei die Verbindung nach Allgemeiner Formel (I) (2R,4R)-4-Methyl-1-((2S)-2-[(3RS)-3-methyl-1,2,3,4-tetrahydrochinolin-8-yl]-sulfonyl]-amino-5-guanidinopentanoyl)-piperidin-2-carbonsäure ist.

**6.** Oberflächenbehandlungsagens für medizinische Geräte oder medizinische Materialien, wobei das Oberflächenbehandlungsagens die hydrophile Polymerverbindung gemäß einem der Ansprüche 1 bis 5, welche eine gerinnungshemmende Aktivität besitzt, umfasst.

**7.** Medizinisches Gerät oder medizinisches Material, welches mit dem Oberflächenbehandlungsagens gemäß Anspruch 6 behandelt ist.

## Revendications

**1.** Composé polymère hydrophile comprenant un composé polymère qui inhibe l'adhésion des plaquettes et un composé qui inhibe la coagulation du sang, lié audit composé polymère, dans lequel
ledit composé polymère qui inhibe l'adhésion des plaquettes est un copolymère de monomères choisis dans le groupe constitué d'éthylène glycol, d'acétate de vinyle, de vinylpyrrolidone, de propylène glycol, d'alcool vinylique et de siloxane, et
ledit composé qui inhibe la réaction de coagulation du sang est un composé représenté par la formule générale (I) :

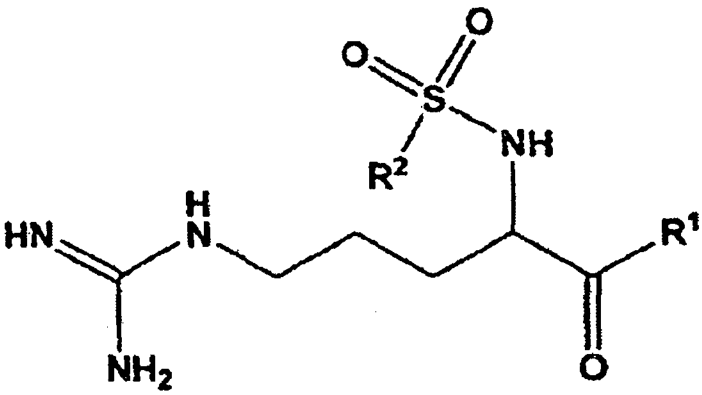

... (I)

dans laquelle $R^1$ représente un groupe (2R,4R)-4-alkyl-2-carboxypipéridino ; $R^2$ représente un groupe phényle ou un résidu de composé polycyclique fusionné, le résidu de composé polycyclique fusionné étant optionnellement substitué par un groupe alkyle inférieur, un groupe alcoxy inférieur ou un groupe amino qui est substitué par un groupe alkyle inférieur.

2. Composé polymère hydrophile selon la revendication 1, dans lequel ledit composé polymère qui inhibe l'adhésion des plaquettes est un copolymère composé d'une macromolécule hydrophobe et d'une macromolécule hydrophile, et a une quantité d'adsorption au poly(méthylméthacrylate) non inférieure à 0,1 pg/$mm^2$.

3. Composé polymère hydrophile selon la revendication 1 ou la revendication 2, dans lequel ledit composé qui inhibe la réaction de coagulation du sang est un composé ayant une activité anti-thrombine.

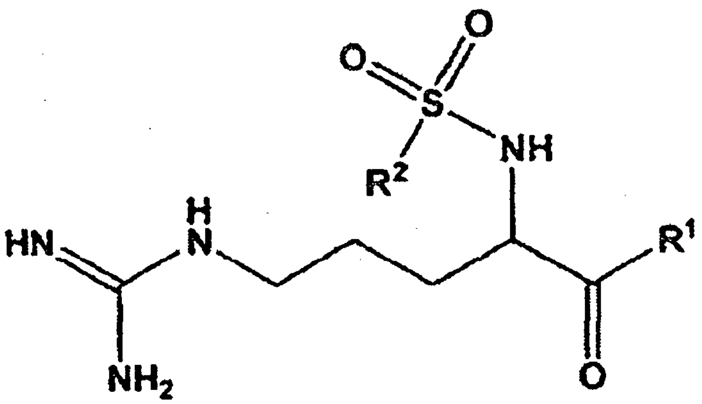

... (I)

4. Composé polymère hydrophile selon l'une quelconque des revendications précédentes, dans lequel ledit copolymère est une silicone modifiée par un polyéther.

5. Composé polymère hydrophile selon la revendication 1 ou la revendication 4, dans lequel ledit composé de formule générale (I) est l'acide (2R,4R)-4-méthyl-1-((2S)-2-[(3RS)-3-méthyl-1,2,3,4-tétrahydroquinoline-8-yl]sulfonyl]amino-5-guanidinopentanoyl)-pipéridine-2-carboxylique.

6. Agent de traitement de surface pour dispositifs médicaux ou équipements médicaux, l'agent de traitement de surface comprenant ledit composé polymère hydrophile selon l'une quelconque des revendications 1 à 5, qui a une activité anticoagulante.

7. Dispositif médical ou équipement médical traité par ledit agent de traitement de surface selon la revendication 6.

【 :1】

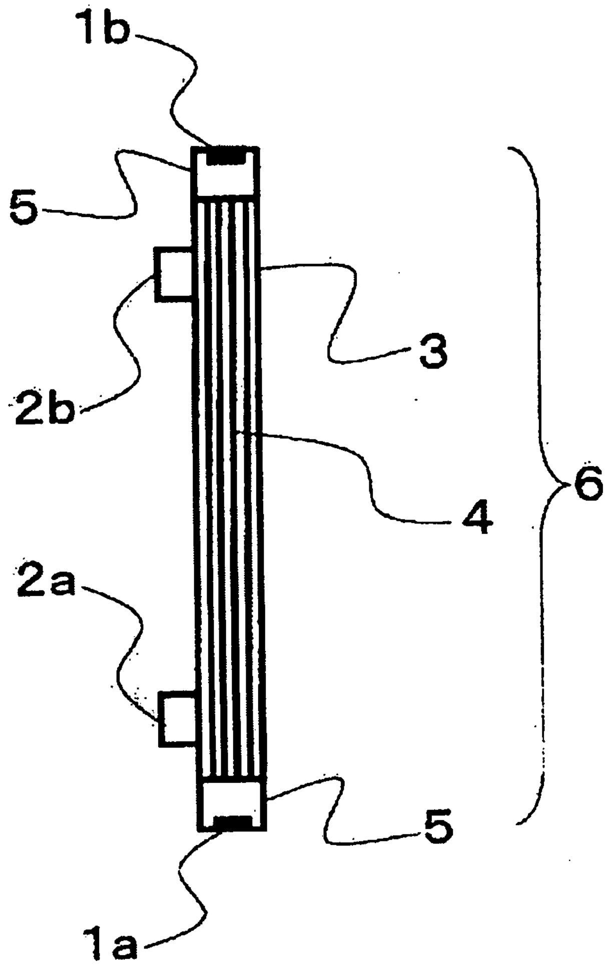

【 2】

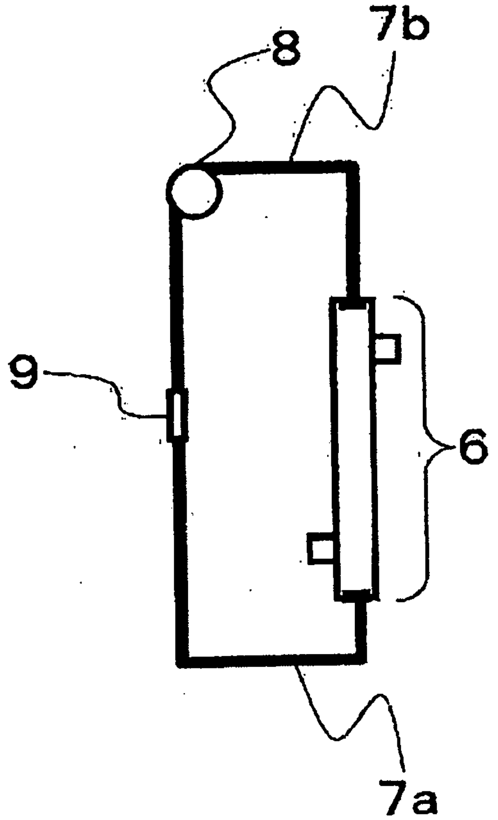


【 3】

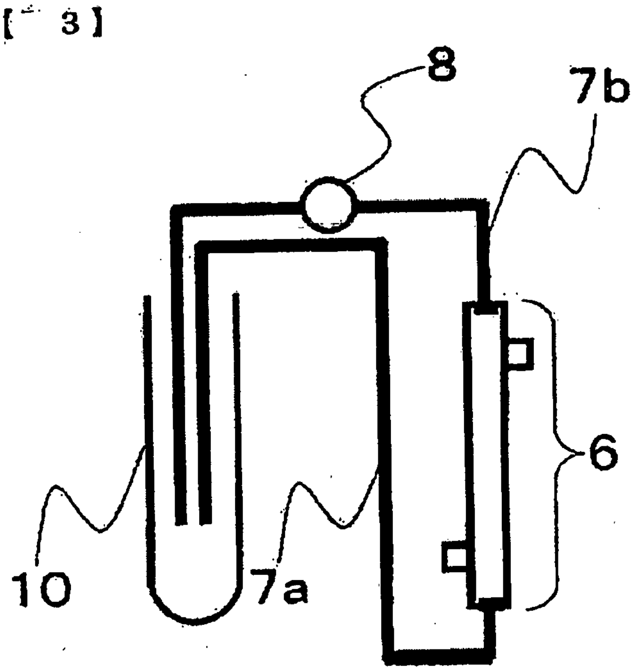

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2003507082 PCT
- JP 2001213984 A
- JP 2004525888 PCT
- JP 2006291193 A
- WO 08032758 A
- JP 2009225824 A
- JP 2010082067 A
- JP 2007181691 A
- JP 2007181692 A


### Non-patent literature cited in the description


- **MASAMITSU KANAI et al.** Clinical Test Handbook. Kanihara shuppan, 1993, vol. 30, 416-418